# DEMANDE DE BREVET EUROPEEN

(11) **EP 0 808 612 A1**
(43) Date de publication de la demande: **26.11.1997**
(21) Numéro de dépôt: 96402581.1
(22) Date de dépôt: 28.11.1996
(51) Int. Cl.: A61F 2/06

(54) **Endoprothèse destinée à être mise en place dans un canal corporel**

(30) Priorité: 24.04.1996 FR 9605153
(71) Demandeur: Legona Anstalt, Vaduz (LI)
(72) Inventeur: Robichon, Manuel, 95840 Villiers Adam (FR); Meunier, Jean-Jacques, 75016 Paris (FR)
(74) Mandataire: Coester, Jacques Charles

(57) **Abrégé**

Endoprothèse intracorporelle réalisée à partir d'au moins un fil ou ruban en alliage à mémoire de forme, caractérisée en ce que les fils ou rubans sont pliés selon des spires successives (1, 2) en forme de boucles à brins (3, 4) sensiblement parallèles reliés entre eux par une partie courbe (5) pour délimiter des fourreaux sensiblement cylindriques reliés les uns aux autres et en ce que des fourreaux d'extrémité au moins présentent une raideur supérieure à celle des autres fourreaux.

## Description

La présente invention a pour objet une endoprothèse destinée à être mise en place dans un canal du corps humain, en particulier dans une artère sténosée ou subissant un anévrisme.

La technique antérieure a déjà fait connaître des implants ou stents en fil métallique et notamment des stents réalisés par un fil métallique continu formé en zigzag puis enroulé en hélice, cette prothèse étant compressible et autodilatable a la suite d'une compression préalable.

Une telle prothèse est décrite dans FR-A-94 024475 qui propose, en outre, de réaliser le fil métallique en alliage à mémoire de forme pour permettre d'exercer une contrainte régulière au moyen de la prothèse.

Une prothèse analogue est décrite également dans EP-A-0 556 850 dans lequel des fils de section quelconque sont réunis pour délimiter un filet cylindrique entouré par un fil, lui-même enroulé suivant une hélice.

Un fil enroulé en hélice et revêtu d'une gaine pour maintenir le fil enroulé en hélice a un diamètre donné est, par ailleurs, décrit dans FR-A-91 00820 pour constituer une endoprothèse vasculaire.

Cette technique est également proposée par EP-A-0 380 668 qui décrit également un cathéter pour la mise en place de l'endoprothèse

Par ailleurs, un fil tout d'abord plié en zigzag puis enroulé pour former des spires hélicoïdales parallèles est décrit pour la réalisation d'une endoprothèse dans EP-A-0 357 003.

Les éléments ci-dessus illustrant l'état antérieur de la technique ne permettent pas jusqu'à présent d'appliquer à la paroi interne d'un canal corporel, plus particulièrement d'une artère, des efforts variables dépendant de l'état pathologique de ce canal tout en faisant que l'endoprothèse soit toujours convenablement maintenue en place sans risque de déplacement ni d'endommagement du canal dans lequel elle se trouve.

Ce résultat est obtenu par l'endoprothèse intracorporelle de l'invention. En outre l'endoprothèse de l'invention rend possible sa mise en place au niveau de l'embranchement de deux artères. D'une façon plus générale, l'invention rend possible le traitement de sténoses périphérique, aortique et thoracique de même que celui d'anévrismes ainsi que celui notamment des voies biliaires, etc.

Conformément à l'invention, l'endoprothèse intracorporelle réalisée à partir d'au moins un fil ou ruban en alliage à mémoire de forme est caractérisée en ce que les fils ou rubans sont pliés selon des spires successives en forme de boucles à brins sensiblement parallèles reliés entre eux par une partie courbe pour délimiter des fourreaux sensiblement cylindriques reliés les uns aux autres et en ce que des fourreaux d'extrémité au moins présentent une raideur supérieure à celle des autres fourreaux.

Suivant une autre caractéristique importante de l'invention, l'alliage à mémoire de forme utilisé pour la réalisation des spires successives de chaque fourreau cylindrique est choisi pour que les brins des fourreaux soient sensiblement parallèles à la température du corps humain.

Diverses autres caractéristiques de l'invention ressortent d'ailleurs de la description détaillée qui suit.

Des formes de réalisation de l'objet de l'invention sont représentées, à titre d'exemples non limitatifs, au dessin annexé.

La fig. 1 est une élévation, en partie schématique, de l'endoprothèse intracorporelle de l'invention.

La fig. 2 est une élévation partielle, à plus grande échelle, illustrant un mode d'assemblage d'éléments de la prothèse de la fig. 1.

La fig. 3 est une élévation partielle, à plus grande échelle, illustrant un autre mode d'assemblage d'éléments de la prothèse de la fig. 1.

La fig. 4 est une coupe, à plus grande échelle, vue suivant la ligne IV-IV de la fig. 2.

La fig. 5 est une coupe, analogue à la fig. 4, d'une variante.

La fig. 6 est une élévation schématique illustrant une caractéristique supplémentaire de la prothèse.

La fig. 7 est une élévation partielle schématique d'une prothèse particulière.

La fig. 8 est une élévation partielle schématique illustrant une caractéristique particulière de réalisation des prothèses.

L'endoprothèse, objet de l'invention, est réalisée à partir de fils ou de rubans en alliage à mémoire de forme, par exemple en alliage métallique nickel/titane, qui est susceptible d'être recuit pour conserver une mémoire thermique correspondant à la forme qui lui est donnée au moment de ce recuit alors que cette forme est modifiée lorsque le fil ou le ruban est soumis à une variation de température, notamment à un refroidissement.

On fait en sorte que l'alliage soit mis en forme pour demeurer stable à une température de l'ordre de celle du corps humain.

Selon l'invention, le fil ou le ruban est plié pour délimiter des spires successives 1, 2 ... n présentant chacune la forme d'une boucle dont les brins 3, 4 sont sensiblement parallèles à la température du corps humain et reliés entre eux par une partie courbe 5.

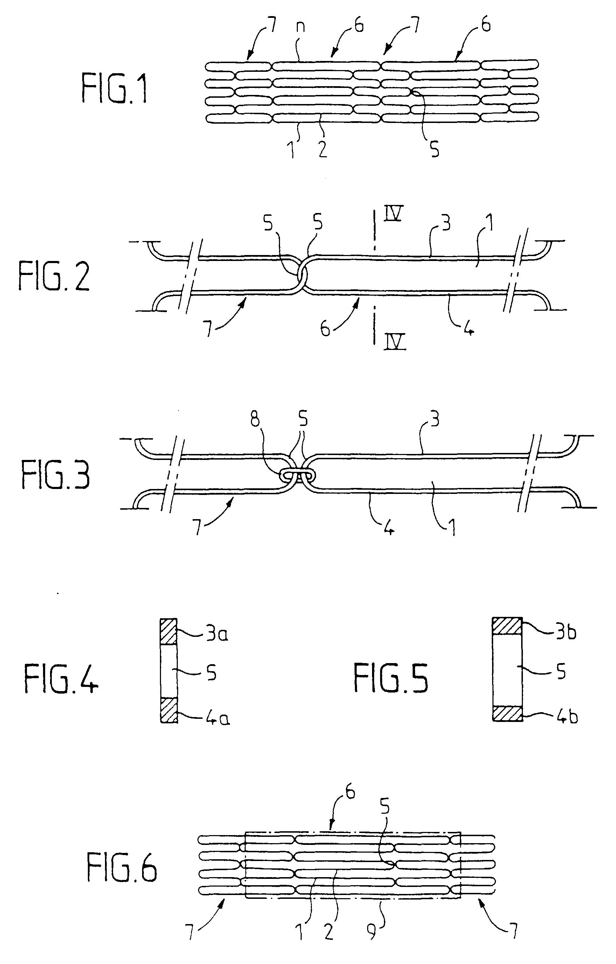

Selon le mode de réalisation de la fig. 1, on réalise successivement des segments distincts 6 et 7 dits dans ce qui suit segments longs pour ceux désignés par 6, et segments courts pour ceux désignés par 7.

Les spires 1, 2 ... n d'un segment, par exemple d'un segment long 6, sont reliées aux spires d'un segment court 7 par tissage, comme illustré à la fig. 2, ou par un lien 8 comme illustré par la fig. 3.

Le lien 8 est, par exemple réalisé par un fil de suture chirurgical non résorbable ou par une agrafe en même matière que celle des fils ou des rubans constitutifs des spires successives de chaque segment. Les segments longs 6 et les segments courts 7 sont mis en forme pour réaliser des fourreaux cylindriques dont les brins sont sensiblement alignés lorsque leurs parties courbes 5 sont réunies par tissage ou au moyen du lien 8.

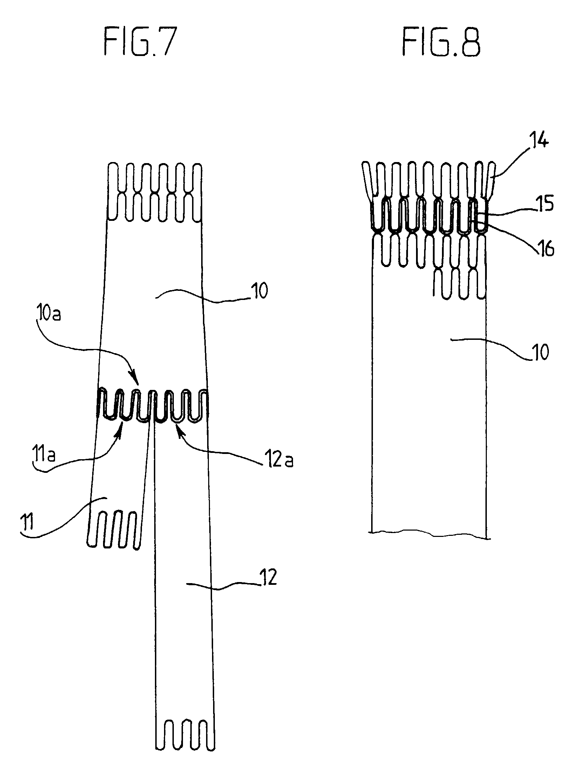

Bien qu'un fil de section ronde d'un diamètre de préférence compris entre 0,20 et 0,50 mm soit utilisable pour la fabrication de l'endoprothèse de l'invention, il est souvent préféré d'utiliser un ruban de segment rectangulaire comme illustré par la fig. 4 qui montre deux brins 3a, 4a d'un ruban de section rectangulaire dont les petits côtés se font face tandis que la fig. 5 montre un ruban, également de section rectangulaire, mais dont les brins 3b, 4b se font face par un de leurs grands côtés. Des rubans de section triangulaire peuvent aussi avantageusement être utilisés.

Outre ce qui précède et ainsi que l'illustre la fig. 1, il est avantageux selon l'invention que deux fourreaux cylindriques réalisés par des segments longs 6 soient séparés par un ou deux fourreaux cylindriques réalisés à partir des segments courts 7 et qu'un ou deux segments cylindriques réalisés par des segments courts prolongent les fourreaux réalisés par les segments longs 6.

L'utilisation de fourreaux réalisés à partir de segments courts a pour effet de raidir la prothèse à ses extrémités et à sa partie médiane, ce qui facilite sa tenue et son maintien en place dans un canal corporel, par exemple dans une artère.

Une prothèse très courte peut, selon l'invention comporter seulement un fourreau long et deux fourreaux courts à ses extrémités. Au contraire, une prothèse longue peut comporter des séries successives de fourreaux longs et de fourreaux courts.

Par exemple, une prothèse devant mesurer 6 cm de long comporte avantageusement deux fourreaux longs reliés directement entre eux et deux fourreaux courts de part et d'autre desdits deux fourreaux longs.

De nombreuses autres combinaisons peuvent être réalisées selon la longueur que doit présenter la prothèse et également selon le diamètre efficace qu'elle doit maintenir à l'intérieur du canal corporel.

La disposition que doit présenter la prothèse selon laquelle des fourreaux longs et des fourreaux courts sont utilisés permet de l'adapter à la pathologie à traiter. En particulier, il est avantageux d'utiliser des fourreaux courts au niveau d'une sténose à traiter alors que des fourreaux longs sont plus appropriés dans des artères présentant une paroi faible.

Dans certains cas et supplémentairement, la prothèse est mise en place dans un manchon 9 illustré à la fig. 6, ce manchon poreux ou non étant évidemment en matière biocompatible et étant destiné à des artères présentant un anévrisme.

Lorsqu'un manchon 9 est prévu, les extrémités constituées par des fourreaux courts permettent d'assurer un maintien convenable de la prothèse de part et d'autre de la zone faible de l'artère. Une partie ou la totalité d'un ou des deux fourreaux cylindriques courts peut dépasser du manchon 9 pour développer une force radiale importante en permettant ainsi d'assurer le maintien en place de la prothèse.

Pour la réalisation du manchon 9, il est possible d'utiliser une matière autre qu'un métal par exemple il est possible d'utiliser un polymère (silicone, gélatine, etc).

La fig. 7 illustre une réalisation particulière d'une prothèse dite bifurquée pour être mise en oeuvre à l'intersection de deux canaux corporels par exemple au niveau de l'aorte abdominale pour l'appareillage de sténoses. La prothèse comporte un fourreau de grand diamètre 10, à boucles tissées ou ligaturées à la manière des fig. 2 ou 3, qui est raccordé à des fourreaux 11 et 12 réalisant la bifurcation.

Le raccord 13 entre les différents fourreaux 10, d'une part, et 11, 12, d'autre part, est réalisé par tissage des segments d'extrémité 10a et 11a, 12a. Une raideur appropriée à la partie confluante des canaux corporels est ainsi obtenue. Les fourreaux 10, 11 et 12 peuvent être réalisés par des segments successifs de même longueur ou par des segments courts et longs comme décrit en référence aux fig. 1 et 6.

La fig. 8 illustre une réalisation permettant de raidir l'extrémité d'un fourreau, par exemple l'extrémité supérieure du fourreau 10 tout en assurant une grande souplesse à la partie terminale. Dans ce cas le segment extrême 14 est constitué par un fil ou ruban unique et le segment précédent par deux fils ou rubans accolés 15, 16 tissés ou ligaturés aux fils ou rubans constituant les segments adjacents. La disposition ci-dessus qui augmente la rigidité en deça du segment terminal 14 a pour effet que ce dernier est légèrement évasé pour assurer une application souple contre la paroi du canal corporel.

Outre ce qui précède, il est avantageux de munir d'au moins un marqueur 17 (fig. 7) au moins un des brins d'un segment. Le marqueur peut être constitué par un fil de platine pour augmenter la radio-opacité d'au moins un endroit de la prothèse.

L'invention n'est pas limitée aux exemples de réalisation, représentés et décrits en détail, car diverses modifications peuvent y être apportées sans sortir de son cadre.

## Revendications

1. Endoprothèse intracorporelle réalisée à partir d'au moins un fil ou ruban en alliage à mémoire de forme, caractérisée en ce que les fils ou rubans sont pliés selon des spires successives (1, 2) en forme de boucles à brins (3, 4) sensiblement parallèles reliés entre eux par une partie courbe (5) pour délimiter des fourreaux sensiblement cylindriques reliés les uns aux autres et en ce que des fourreaux d'extrémité au moins présentent une raideur supérieure à celle des autres fourreaux.

2. Endoprothèse suivant la revendication 1, caractérisée en ce que les spires successives sont fabriquées à partir de fils ou de rubans.

3. Endoprothèse suivant l'une des revendications 1 et 2, caractérisée en ce que les brins successifs (3, 4) sont de section rectangulaire ou triangulaire.

4. Endoprothèse suivant l'une des revendications 1 à 3, caractérisée en ce que les brins successifs (3, 4) présentent une section de l'ordre de 0,20 à 0,50 mm.

5. Endoprothèse suivant l'une des revendications 1 à 4, caractérisée en ce que l'alliage à mémoire de forme utilisé pour la réalisation des spires successives de chaque fourreau cylindrique est choisi pour que les brins (3, 4) soient sensiblement parallèles à la température du corps humain.

6. Endoprothèse suivant l'une des revendications 1 à 5, caractérisée en ce que des fourreaux cylindriques de plus grande raideur sont mis en place près des extrémités de la prothèse.

7. Endoprothèse suivant l'une des revendications 1 à 5, caractérisée en ce qu'elle est constituée par une succession de fourreaux cylindriques formant des segments longs (6) et des segments courts alternés (7) reliés entre eux.

8. Endoprothèse suivant l'une des revendications 1 à 7, caractérisée en ce que les segments longs (6) et les segments courts (7) sont constitués par des spires successives (1, 2 ... n) formées chacune par deux brins (3, 4) reliés par une partie courbe (5), lesdits brins (3, 4) étant paralléles à la température du corps.

9. Endoprothèse suivant l'une des revendications 1 à 8, caractérisée en ce qu'elle comporte un fourreau de grand diamètre (10) raccordé à des fourreaux de petit diamètre (11, 12) pour son application à la partie confluante de canaux corporels.

10. Endoprothèse suivant l'une des revendications 1 à 9, caractérisée en ce que la raideur des fourreaux est augmentée en accolant deux fils (15, 16) sur un segment.

11. Endoprothèse suivant l'une des revendications 1 à 10, caractérisée en ce que des jeux de deux fourreaux courts sont mis en place aux extrémités de la prothèse.

12. Endoprothèse suivant l'une des revendications 1 à 11, caractérisée en ce que les fourreaux assemblés sont disposés dans un manchon (9).

13. Endoprothèse suivant la revendication 12, caractérisée en ce que des fourreaux courts font saillie du manchon (9).

14. Endoprothèse suivant l'une des revendications 1 à 13, caractérisée en ce qu'elle comporte au moins un marqueur augmentant la radio-opacité.

15. Endoprothèse sensiblement telle que décrite et représentée aux dessins annexés.
